(19) Europäisches Patentamt
European Patent Office
Office européen des brevets

(11) Veröffentlichungsnummer : **0 210 137 B1**

(12) # EUROPÄISCHE PATENTSCHRIFT

(45) Veröffentlichungstag der Patentschrift : **11.09.91 Patentblatt 91/37**

(51) Int. Cl.$^5$ : **C07D 513/04**, A01N 43/90, // (C07D513/04, 285:00, 249:00)

(21) Anmeldenummer : **86810328.4**

(22) Anmeldetag : **18.07.86**

(54) **Anellierte Triazol-Verbindungen.**

Die Akte enthält technische Angaben, die nach dem Eingang der Anmeldung eingereicht wurden und die nicht in dieser Patentschrift enthalten sind.

(30) Priorität : **24.07.85 CH 3222/85**
**30.08.85 CH 3743/85**

(43) Veröffentlichungstag der Anmeldung : **28.01.87 Patentblatt 87/05**

(45) Bekanntmachung des Hinweises auf die Patenterteilung : **11.09.91 Patentblatt 91/37**

(84) Benannte Vertragsstaaten : **BE CH DE FR GB IT LI NL**

(56) Entgegenhaltungen :
**EP-A- 0 075 267**
**EP-A- 0 104 484**
**FR-A- 2 322 148**

(73) Patentinhaber : **CIBA-GEIGY AG**
**Klybeckstrasse 141**
**CH-4002 Basel (CH)**

(72) Erfinder : **Böhner, Beat, Dr.**
**Hügelweg 3**
**CH-4102 Binningen (CH)**
Erfinder : **Pissiotas, Georg, Dr.**
**Am Sonnenrain 71**
**W-7850 Lörrach (DE)**
Erfinder : **Moser, Hans, Dr.**
**Maispracherstrasse 12**
**CH-4312 Magden (CH)**

EP 0 210 137 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue anellierte Triazol-Verbindungen der untenstehenden Formel I mit herbizider und den Pflanzenwuchs regulierender Wirkung, sowie die Herstellung dieser neuen Verbindungen. Ferner betrifft sie die Mittel, welche die neuen Verbindungen enthalten sowie deren Verwendung zur selektiven Bekämpfung von Unkräutern oder zur Regulierung des Pflanzenwuchses.

Die neuen anellierten Triazol-Verbindungen entsprechen der Formel I

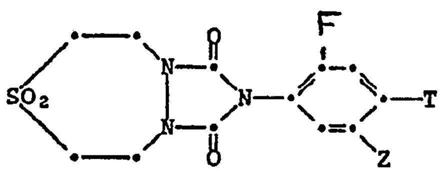

(I)

worin

T Halogen und

Z einen Rest -XR oder -COXR

X Sauerstoff, Schwefel oder den Rest $-NR_1-$,

R Wasserstoff, einen $C_1$-$C_6$-Alkyl-, $C_3$-$C_6$-Cycloalkyl-, einen über ein gesättigtes Kohlenstoffatom gebundenen $C_3$-$C_6$- Alkenyl- oder $C_3$-$C_6$-Alkinylrest der unsubstituiert oder durch Halogen substituiert ist, oder einen Rest -A- $COXR_1$ wobei

A eine $C_1$-$C_4$ Alkylenbrücke bedeutet und

$R_1$ Wasserstoff oder $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeuten.

In der Formel I ist unter Halogen, Fluor, Chlor, Brom oder Iod zu verstehen. Beim Substituenten T ist Chlor und Brom bevorzugt.

Der Ausdruck Alkyl allein oder als Teil eines Substituenten umfasst verzweigte oder geradkettige Alkylgruppen. Beispiele sind Methyl, Aethyl, n-Propyl, Isopropyl, n-Butyl, Isobutyl, sek.-Butyl, tert.-Butyl, n-Pentyl, Isopentyl, Neopentyl sowie Hexyl mit seinen Isomeren.

Die Alkenyl- und Alkinylreste sind über ein gesättigtes Kohlenstoffatom gebunden und können ebenfalls verzweigt oder geradkettig sein und umfassen vorzugsweise 3-6 Kohlenstoffatome. Beispiele sind Allyl, Methallyl, Butenyl, Butadienyl, Methylbutenyl, Dimethylbutenyl, Pentenyl, Hexenyl, Pentendienyl und Hexendienyl, Butin, Methylbutin, Pentin, Hexin, Dimethylbutin.

Die dem Rest A entsprechende Alkenylbrücke kann geradkettig oder verzweigt sein. In Frage kommen beispielsweise Methylen, Aethylen, 1-Methylmethylen, 1,1-Dimethyläthylen, 1- oder 2-Methyläthylen, 1,2-Dimethyläthylen, 1,1- oder 2,2-Dimethyläthylen, Propylen oder Butylen.

Die Verbindungen der Formel I haben pflanzenwuchsregulierende Wirkung und können zur Hemmung des vegetativen Wachstums verwendet werden.

Hauptsächlich haben die Verbindungen der Formel I jedoch herbizide Wirkung und eignen sich zur Unkrautbekämpfung, besonders von dikotylen Unkräutern. Dabei hat es sich herausgestellt, dass mit diesen Verbindungen die sonst sehr resistenten Problemunkräuter der Familie Galium, Labkrautgewächse, wie z.B. Galium verum, echtes Labkraut, Galium aparine, Kleblabkraut, Galium mollugo, gemeinsames Labkraut etc., gegen welche andere bekannte Herbizide oft nur eine ungenügende Wirkung zeigen, vernichtet werden können.

Ein grosser Vorteil ist dabei, dass die neuen Verbindungen der Formel I sich gegenüber vielen Kulturpflanzen, wie Getreide , Mais oder Reis, aber auch Raps selektiv verhalten und zur Unkrautbekämpfung in solchen Kulturen eingesetzt werden können.

Die Wirkstoffe der Formel I werden in der Regel mit Aufwandmengen von 0,005 bis 4 kg/ha insbesondere 0,001 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren -

sehr geringen Aufwandmengen.

Aus der DOS 26 38 543 sind bereits 9-Aryl-8,10-dioxo-4,1,7,9-thiatriazabicyclo[5.3.0]-decan-4,4-dioxide als Herbizide bekannt geworden. Aus den EP-A 75,267 und EP-A 104,484 sind in 1,2-Stellung annelierte 4-Phenyl-3,5-dion-1,2,4-triazole mit herbizider Wirkung bekannt, die im Ringsystem keine Sulfolylgruppe enthalten.

Ueberraschenderweise wurde nun gefunden, dass die neuen Verbindungen der Formel I wesentlich stärkere herbizide Eigenschaften besitzen als die vorbekannten Verbindungen. Mit den neuen Verbindungen der Formel I kann deshalb die Menge Aktivsubstanz pro Hektare drastisch gesenkt werden, was sowohl oekologisch und oekonomisch von Vorteil ist.

Von den neuen anellierten 1-Phenyl-2,5-dioxo-1H-1,3,4-triazol Verbindungen der Formel I zeigten diejenigen gute Wirkung, in denen

T Fluor, Chlor oder Brom bedeuten während
Z die unter der Formel I gegebene Bedeutung hat, insbesondere diejenigen, in denen
T Chlor oder Brom,
Z einen Rest -XR oder -COXR
X Sauerstoff, Schwefel, den Rest -$NR_1$-
R Wasserstoff, $C_1$-$C_4$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl und
$R_1$ Wasserstoff oder $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeuten.

Ebenfalls gute Wirkung zeigten die Verbindungen der Formel I, in denen

T Chlor oder Brom
Z einen Rest -XR,
X Sauerstoff oder Schwefel,
R Wasserstoff, einem $C_1$-$C_6$ Alkyl-, $C_3$-$C_6$ Cycloalkyl-, einen über ein gesättigtes Kohlenstoffatom gebundenen $C_3$-$C_6$ Alkenyl- oder $C_3$-$C_6$ Alkinylrest bedeutet, der unsubstituiert oder durch Halogen substituiert ist, ferner diejenigen in denen
T Chlor oder Brom,
Z einen Rest -XR,
X einen Rest -$NR_1$-
R Wasserstoff, $C_1$-$C_6$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl und
$R_1$ Wasserstoff, $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeutet.

Weiterhin zeigen die Verbindungen, in denen

T Chlor oder Brom,
Z einen Rest -COXR
X Sauerstoff oder Schwefel und
R Wasserstoff, $C_1$-$C_6$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl bedeuten, ferner diejenigen in denen
T Chlor oder Brom
Z einen Rest XR
R einen Rest -A-$COXR_1$
A eine $C_1$-$C_4$ Alkylenkette
X Sauerstoff oder Schwefel und
$R_1$ Wasserstoff, $C_1$-$C_6$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl bedeuten,

insbesonders

9-(2-Fluor-4-chlor-5-isopropyloxy-phenyl)-8,10-dioxo-4,1,7,9-thiatriazabicyclo[5.3.0]-decan-4,4-dioxid

gute Wirkung.

Die Verbindungen der Formel I werden dadurch hergestellt, indem man ein 1-(2-Phenyl)-1H-1,3,4-triazolidin-2,5-dion der Formel II

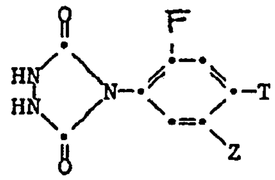

II

worin Q, T und Z die unter der Formel I gegebene Bedeutung haben in einem inerten organischen Lösungsmittel bei einer Temperatur von 0° bis 150°C in Gegenwart der Katalytischen Menge einer Base mit Divinylsulfon $(CH_2=CH)SO_2$ umsetzt.

Diese Kondensation oder Addition wird am besten in einem polaren aprotischen organischen Lösungsmittel vorgenommen, in Gegenwart von etwas Base als Katalysator. Siehe dazu G. Zinner Arch. Pharm. 299 (1966) Seite 312-314, worin z.B. die Herstellung von 9-Phenyl-8,10-dioxo-4,1,7,9-thiatriazabicyclo[5.3.0]decan-4,4-dioxid beschrieben ist oder auch die DE-A-2 638 543, wo die Herstellung von 9-Aryl-8,10-dioxo oder 8-on-10-thion-4,1,7,9-thiatriazabicyclo-[5.3.0]decan-4,4-dioxiden allgemein beschrieben ist.

Polare aprotische organische Lösungsmittel, welche für diese Kondensation in Frage kommen, sind z.B. Formamide, Aralkylsulfoxide, cyclische Ketone, niedere Alkanole oder Nitrile.

Diese Kondensations- oder Additionsreaktion wird durch Beigabe von etwas Base, wie alkanolischer KOH oder NaOH katalysiert. Die Temperatur kann dabei zwischen O°C bis 150°C variieren.

Die Ausgangsstoffe der Formel II sind zu einem grossen Teil neu. Die Herstellung des 2-Phenyl-1,2,4-triazolidin-3,5-dions, auch 4-Phenylurazol oder N-Phenylimid der Azodicarbonsäure genannt, wurde bereits von J. Thiele et al. Ann. 283 (1894) S. 1 beschrieben. Weitere Literaturstellen dazu sind J. Stolle Ber. 45 (1912) S. 273, G. Zinner et al. Arch. Pharm. 294 (1961) S. 370-372 sowie R. C. Cockson et al. Tetrahedron Letters 14 (1962), S. 615-618. Man lässt dazu z.B. ein im Phenylkern entsprechend substituiertes 1-Aethoxycarbonyl-4-(phenyl)-semicarbazid, $C_2H_5$-O-CO-NH-NH-CO-NH-phenyl, in heissem alkalischen Milieu ringschliessen und isoliert das 4-(2-Phenyl)-1,2,4-triazolidin-3,5-dion dann aus saurem Milieu, aus dem es kristallin ausfällt.

Das 1-Aethoxycarbonyl-4-phenyl-semicarbazid oder ähnliche für die Herstellung der 1,3,4-Triazol-2,5-dion der Formel II benötigte Semicarbazide Formel III

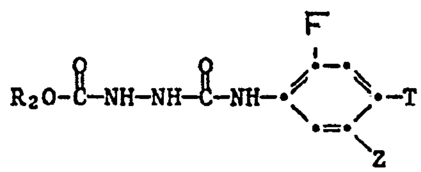

(III)

worin $R_2$ einen $C_1$-$C_6$ Alkyl oder den Benzylrest und T und Z die unter der Formel I gegebene Bedeutung haben, können durch Umsetzen von Hydrazinhydrat mit der äquimolaren Menge eines Dialkylcarbonates und Umsetzen des so entstandenen Alkylcarbazates mit der äquimolaren Menge eines Phenylisocyanates erhalten werden.

Die Herstellung der neuen anellierten Triazol-Verbindungen der Formel I kann durch das folgende Formelschema wiedergegeben werden:

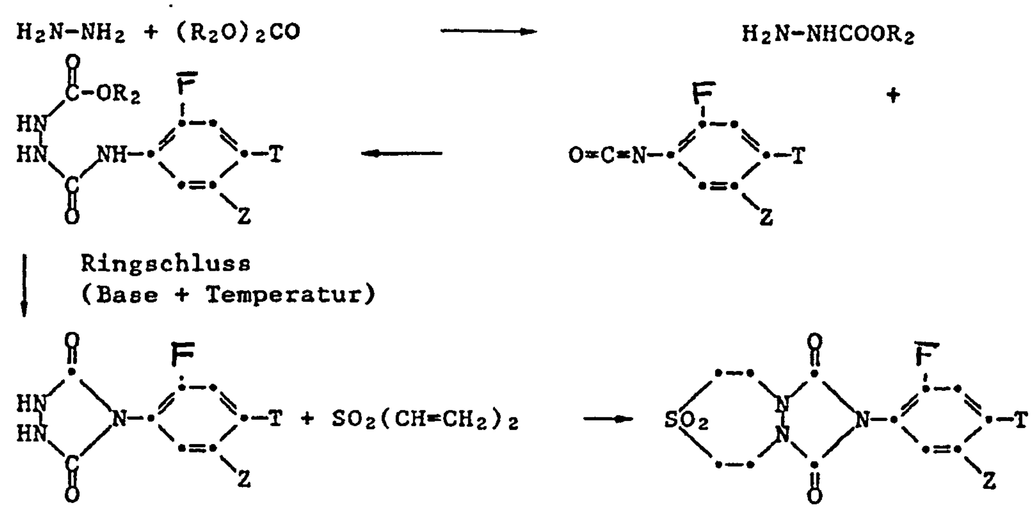


II I

Die Verbindungen der Formel I können entsprechend der Bedeutung von Z ineinander übergeführt werden. Eine wichtige Rolle kommt dabei den 3-Aminoaniliden, 9-(3-Amino-4-halophenyl)-8,10-dioxo-4,1,7,9-thiatriazabicyclo[5.3.0]-decan-4,4-dioxiden der Formel Ia zu. Sie werden erhalten, indem man ein 9-(4-Halophenyl)-8,10-dioxo-4,1,7,9-thiatriazabicyclo[5.3.0]decan-4,4-dioxid mit 1 bis 1,5 molaren Aequivalenten eines Nitrierungsmittels wie z.B. rauchender Salpetersäure oder einem Gemisch rauchender Salpetersäure/Schwefelsäure behandelt. Als Lösungsmittel kann dazu konzentrierte Schwefelsäure verwendet werden, die Reaktionstemperatur soll zwischen -5° und +5° gehalten werden. Das erhaltene 3-Nitroanilid 9-(3-Nitro-4-halophenyl)-8,10-dioxo-4,1,7,9-thiatriazabicyclo[5.3.0]decan-4,4-dioxid wird dann zum Amin reduziert, z.B. in einem inerten organischen Lösungsmittel mit Wasserstoff bei Normaldruck in Gegenwart von Raney Nickel, oder in Wasser, Essigsäure, Aethanol oder Aethylacetat mit 2,5 bis 10 Moläquivalent Eisenfeilspänen und der mindestens äquimolaren Menge Eisessig bei einer Temperatur von 50-100°.

Diese Umsetzungen können durch folgendes Reaktionsschema dargestellt werden

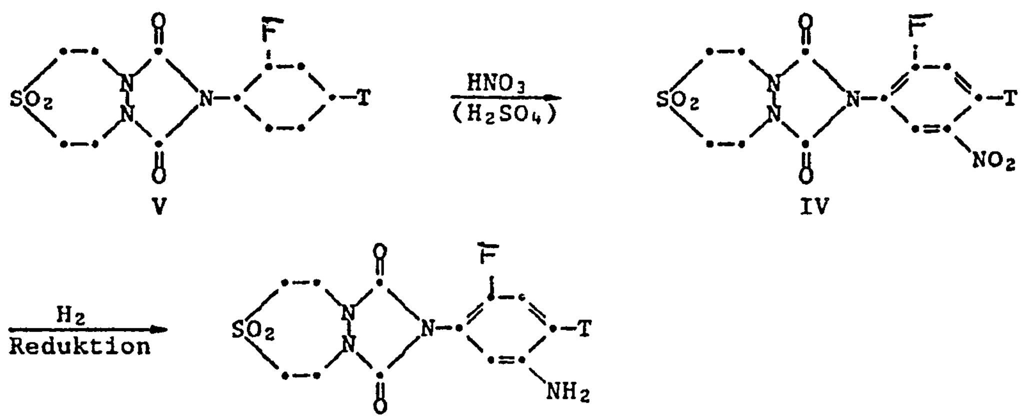


Ia

In diesen Formeln hat T die unter der Formel I gegebene Bedeutung. Die erfindungsgemässen 3-Aminoacetanilide der Formel Ia sind wichtige Schlüsselprodukte für die Herstellung weiterer Verbindungen der Formel I. Sie und die 3-Nitroanilide der Formel IV sind ebenfalls ein Gegenstand dieser Erfindung.

Die 3-Aminoanilide der Formel Ia können in weiteren Verbindungen der Formel I übergeführt werden, z.B. durch Behandeln mit einem Alkylierungsmittel wie einem Alkylhalogenid oder einer Alkylsulfon- oder -arylsulfonsäure gelangt man zu den Verbindungen der Formel I in denen Z als XR einen Aminorest $-N(R)R_1$ verkörpert.

Ferner können die 3-Aminoanilide der Formel Ia in einem Ueberschuss an Mineralsäure (z.B. Salzsäure oder Schwefelsäure mit 1-15 Moläquivalent Natriumnitrit in das Diazoniumsalz übergeführt werden, welches dann durch Zersetzen in Wasser oder Essigsäure mit Kupfercyanat zum 3-Cyanoanilid, 9-(3-Cyano-4-halophenyl)-8,10-dioxo-4,1,7,9-thiatriazabicyclo[5.3.0]decan-4,4-dioxid der Formel VI umgewandelt wird, welches seinerseits durch Kochen mit Wasser und der katalytischen Menge einer Base zum 3-Carboxylanilid, 9-(3-Carboxy-4-halophenyl)-8,10-dioxo-4,1,7,9-thiatriazabicyclo[5.3.0]-decan-4,4-dion der Formel VI hydrolisiert wird. Durch Umsetzen dieses Carbonsäureesters mit einem starken Halogenierungsmittel wie Phosphoroxychlorid, Phosphoroxybromid, Thionylchlorid, Thionylbromid, Sulfurylchlorid oder Bromsuccinimid gelangt man zum Säurehalogenid der Formel VII, welches durch Umsetzen mit einem Alkanol oder Thiol der Formel HXR in eine Verbindung der Formel I übergeführt werden kann, in der Z durch den Carbonsäurerest -COXR verkörpert ist. Diese Umsetzungen können durch folgendes Reaktionsschema dargestellt werden:

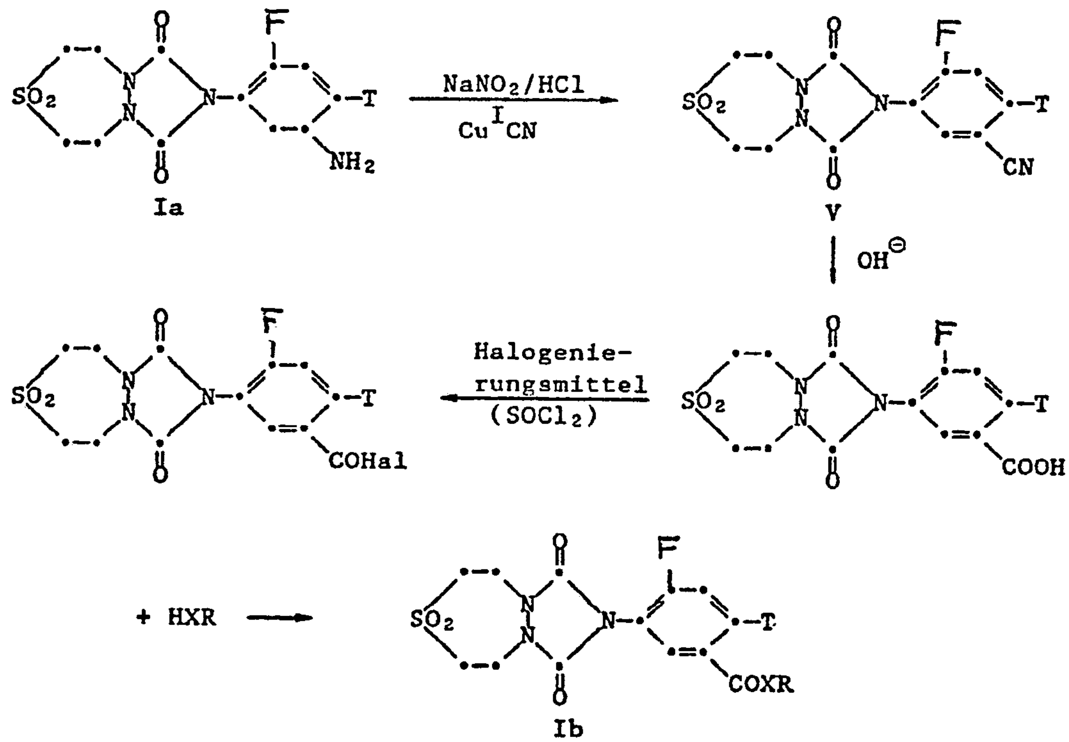


Ia, V, Ib

In diesen Formeln haben R, T und X die unter der Formel I gegebene Bedeutung.

Das durch Reaktion des 3-Aminoanilides der Formel Ia mit Natriumnitrit hergestellte Diazoniumsalz kann entweder zum Phenol hydrolysiert oder in ein Thiophenol bzw. Thiophenol-Derivat überführt werden, wobei diese Derivate ihrerseits wieder veräthert werden können entsprechend den folgenden Gleichungen.

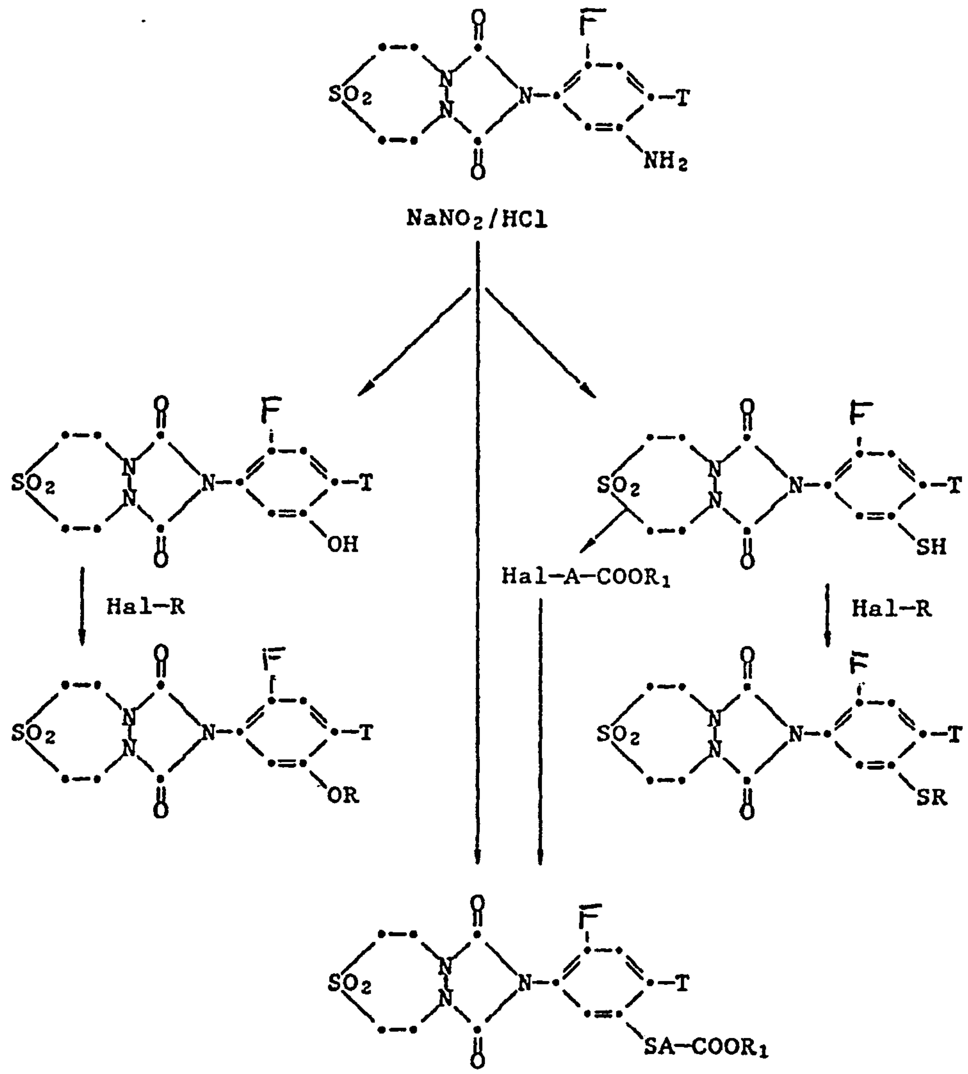


In diesen Formeln haben A, R, $R_1$ und T die unter der Formel I gegebene Bedeutung.

Die Verbindungen der Formel I haben herbizide und starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kultur pflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Die Dessiccations- und Defoliationswirkung dieser Verbindungen wird benutzt um bei Kulturen wie Kartoffeln und Baumwolle bei deren Reife nicht benötigte vegetative Pflanzenteile auszutrocknen um dadurch die Ernte zu erleichtern.

Hauptsächlich haben die Verbindungen der Formel I jedoch herbizide Wirkung und eignen sich zur Unkrautbekämpfung, besonders von dikotylen Unkräutern. Dabei hat es sich herausgestellt, dass mit diesen Verbindungen die sonst sehr resistenten Problemunkräuter der Familie Galium, Labkrautgewächse, wie z.B. Galium verum, echtes Labkraut, Galium aparine, Kleblabkraut, Galium mollugo, gemeinsames Labkraut etc., gegen welche andere bekannte Herbizide oft nur eine ungenügende Wirkung zeigen, vernichtet werden können.

Ein grosser Vorteil ist dabei, dass die neuen Verbindungen der Formel I sich gegenüber vielen Kulturpflanzen, wie Getreide , Mais oder Reis, aber auch Raps selektiv verhalten und zur Unkrautbekämpfung in solchen

Kulturen eingesetzt werden können.

Die Wirkstoffe der Formel I werden in der Regel mit Aufwandmengen von 0,005 bis 4 kg/ha insbesondere 0,001 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Planzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als such wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen.

Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

**Emulgierbare Konzentrate:**

| | | |
|---|---|---|
| Wirkstoff der Formel I: | 1 bis 20 % bevorzugt | 5 bis 10 % |
| oberflächenaktives Mittel: | 5 bis 30 %,vorzugsweise | 10 bis 20 % |
| flüssiges Trägermittel: | 50 bis 94 %,vorzugsweise | 70 bis 85 %. |

**Stäube:**

| | | |
|---|---|---|
| Wirkstoff der Formel I: | 0,1 bis 10 %, vorzugsweise | 0,1 bis 1 % |
| festes Trägermittel: | 99,9 bis 90 %, vorzugsweise | 99,9 bis 99 %. |

**Suspensions-Konzentrate:**

| | | |
|---|---|---|
| Wirkstoff der Formel I: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 %. |

Benetzbare Pulver:

| | | |
|---|---|---|
| Wirkstoff der Formel I: | 0,5 bis 90 %, | vorzugsweise 1 bis 80 % |
| oberflächenaktives Mittel: | 0,5 bis 20 %, | vorzugsweise 1 bis 15 % |
| festes Trägermittel: | 5 bis 95 %, | vorzugsweise 15 bis 90 %. |

Granulate:

| | | |
|---|---|---|
| Wirkstoff der Formel I: | 0,5 bis 30 %, | vorzugsweise 3 bis 15 % |
| festes Trägermittel: | 99,5 bis 70 %, | vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung einiger Verbindungen der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

Beispiel 1: Herstellung von
9-(2-Fluor-4-chlor-5-isopropyloxyphenyl)-8,10-dioxo-4,1,7,9-thiatriazabicyclo[5.3.0]-decan-4,4-dioxid

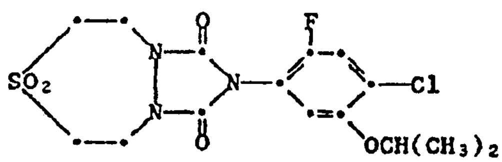

Eine Lösung von 45,0 g (0,195 mol) 2-Fluor-4-chlor-5-isopropyloxy-phenylisocyanat in 50 ml Toluol wird bei 10°C zu einer Lösung von 20,5 g (0,195 mol) Hydrazincarbonsäure-äthylester in 200 ml Toluol getropft. Nach einer Stunde filtriert man den ausgefallenen Niederschlag ab und behandelt diesen mit 250 ml 4N Natronlauge. Man erwärmt auf 60°C und erhält eine klare, gelbe Lösung. Beim Ansäuern auf pH 1 mit konzentrierter Salzsäure fällt ein weisser Niederschlag aus. Nach 1 Stunde Rühren bei 60°C filtriert man ab und trocknet den Rückstand über Phosphorpentoxid. Es werden so 52,5 g (93,8 % der Theorie) 4-(2-Fluor-4-chlor-5-isopropyloxyphenyl)-urazol mit einem Schmelzpunkt von 193 - 194°C erhalten.

17,5 g (0,061 mol) des obigen Urazols werden in 300 ml Aethanol gelöst. Nach Zugabe von 7,2 g (0,061 mol) Divinylsulfon und 0,5 ml 6N Natronlauge heizt man für 8 Stunden am Rückfluss, wobei das Produkt bereits nach ca. 1 Stunde auszufallen beginnt. Man erhält so 23,0 g (92,7 % der Theorie) 9-(2-Fluor-4-chlor-5-isopropyloxy-phenyl-8,10-dioxo-4,1,7,9-thiatriazabicyclo[5.3.0]-decan-4,4-dioxid mit einem Schmelzpunkt von 195 - 196°C.

Beispiel 2: Herstellung von
9-(2-fluor-4-chlor-4-difluormethoxyphenyl)-8,10-dioxo-4,1,7,9-thiatriazabicyclo[5.3.0]-decan-4,4-dioxid

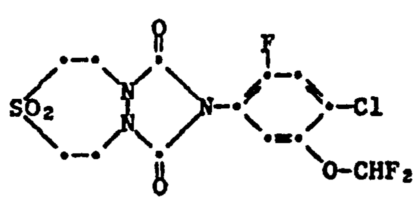

Zu einem auf 50° erwärmten Gemisch von 5,75 g (0,02 Mol) 4-(2-Fluor-4-chlor-5-difluormethoxyurazol und 100 ml Aethanol gibt man 4 Tropfen 6N KOH und 2,36 g (0,02 Mol) Divinylsulfon. Man rührt bis alles in Lösung

geht und erhitzt dann die Lösung 8 Stunden unter Rückfluss. Nach dem Erkalten wird die ausgefallene Substanz abgenutscht und unter Vakuum getrocknet. Man erhält so 6,9 g 9-(2-fluor-4-chlor-5-difluormethoxyphenyl)-8,10-dioxo-4,1,7,9-thiatriazabicyclo[5.3.0]decan-4,4-dioxid, welches ein Schmelzpunkt von 195-196° hat.

Beispiel 3: Herstellung von
9-(2-Fluor-4-chlor-5-aminophenyl)-8,10-dioxo-4,1,7,9-thiadiazabicyclo[5.3.0]decan-4,4-dioxid

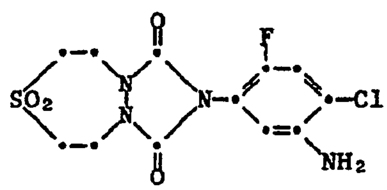

In ein Gesmisch von 15 g 9-(2-Fluor-4-chlor-5-nitro-phenyl)-8,10-dioxo-4,1,7,9-thiatriazabicyclo[5.3.0]decan-4,4-dioxid, 3 g Raney-Nickel, 300 ml Tetrahydrofuran und 150 ml Dimethylformamid wird bei 20-25°C und unter normalem Druck Wasserstoff eingeleitet, bis die stöchiometrische Menge aufgenommen ist. Nach Beendigung der Reaktion wird der Katalysator abfiltriert und das Filtrat unter Vakuum eingedampft. Der Rückstand besteht aus 11,6 g 9-(2-Fluor-4-chlor-5-amino-phenyl)-8,10-dioxo-4,1,7,9-thiadiazabicyclo[5.3.0]decan 4,4-dioxid mit einem Schmelzpunkt von 219-220°C.

Das als Ausgangsprodukt benötigte 9-(2-Fluor-4-chlor-5-nitro-phenyl)-8,10-dioxo-4,1,7,9-thiatriazabicyclo[5.3.0]decan-4,4-dioxid wird wie folgt erhalten:

a) Man gibt in kleinen Portionen 19,1 g 9-(2-Fluor-4-chlor-phenyl)-8,10-dioxo-4,1,7,9-thiatriazabicyclo[5.3.0]decan-4,4-dioxid zu 125 ml konzentrierter Schwefelsäure welche bei 0°C gerührt werden. Zu dieser Lösung tropft man dann unter Rühren und bei 0° 2,5 ml rauchende Salpetersäure. Nach einstündigem Rühren bei Raumtemperatur wird das Reaktionsgemisch auf Eis gegossen und die ausgefallene Masse abgenutscht. Nach dem Waschen mit Wasser und Trocknen erhält man 18 g 9-(2-Fluor-4-chlor-5-nitro-phenyl)-8,10-dioxo-4,1,7,9-thiatriazabicyclo-[5.3.0]-decan-4,4-dioxid mit einem Schmelzpunkt von 251-252°C.

Beispiel 4: Herstellung von
9-(2-Fluor-4-chlor-5-hydroxycarbonyl-methylthiophenyl)-8,10-dioxo-4,1,7,9-thiatriazabicyclo[5.3.0]decan-4,4-dioxid

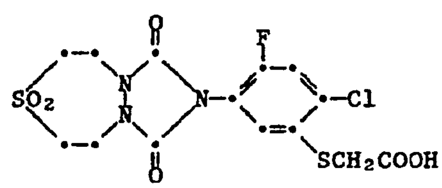

Zu einer Mischung von 17 g (0,047 Mol) 9-(5-Amino-2-fluor-4-chlor-phenyl)-8,10-dion-4,1,7,9-thiatriazabicyclo[5.3.0]decan-4,4-dioxid (Beispiel 3) in 25 ml konz. Schwefelsäure und 130 ml Wasser wird unter langsamem Rühren bei einer Temperatur von -5 - 0° eine Lösung von 3,9 g (0,0565 Mol) Natriumnitrit in 15 ml Wasser unter die Oberfläche zugegeben. Nachdem alles zugegeben ist, wird während einer halben Stunde bei dieser Temperatur weitergerührt, dann nimmt man zuerst das überschüssige Nitrit mit Sulfaminsäure ($H_2NSO_3H$) zurück und gibt dann eine Mischung von 5,5 g (0,06 Mol) Thioglykolsäure und 3,1 g basisches Kupfercarbonat ($2\ CuCO_3 \cdot Cu(OH)_2$) in 50 ml Wasser tropfenweise zu. Man rührt das Gemisch während zwei Stunden bei Raumtemperatur und extrahiert es dann mit Aethylacetat. Die Extrakte werden mit Wasser und gesättigter Salzsole gewaschen, über Natriumsulfat getrocknet und eingedampft. Man erhält so 11,6 g Titelprodukt.

Entsprechend diesen Beispielen werden die in den folgenden Tabellen enthaltenen Verbindungen hergestellt.

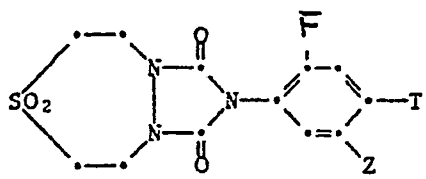

Tabelle 1

| No. | T | Z | |
|---|---|---|---|
| 1001 | Cl | $OCH(CH_3)_2$ | Smp. 195 - 196° |
| 1002 | Cl | $OCH_3$ | Smp. 212 - 214° |
| 1003 | Cl | $OC_2H_5$ | |
| 1004 | Cl | $OC_3H_7n$ | |
| 1005 | Cl | $OC_4H_9n$ | |
| 1006 | Cl | $OCH_2CH(CH_3)_2$ | |
| 1007 | Cl | $OCH(CH_3)C_2H_5$ | |
| 1008 | Cl | $OC(CH_3)_3$ | |
| 1009 | Br | $OCH(CH_3)_2$ | |
| 1010 | Br | $OCH_3$ | |
| 1011 | Cl | $OCH_2CH=CH_2$ | |
| 1012 | Cl | $OCH_2C(CH_3)=CH_2$ | |
| 1013 | Cl | $OCH_2C\equiv CH$ | |
| 1014 | Cl | OH | |
| 1015 | Cl | $SCH_3$ | |
| 1016 | Cl | $SC_2H_5$ | |
| 1017 | Cl | $SCH(CH_3)_2$ | |
| 1018 | Cl | $SC_3H_7n$ | |
| 1019 | Cl | $SC_4H_9n$ | |
| 1020 | Cl | SH | |
| 1021 | Cl | $N(CH_3)_2$ | |
| 1022 | Cl | $NHCH_3$ | |
| 1023 | Cl | $N(CH_3)CH(CH_3)_2$ | |
| 1024 | Br | $N(CH_3)_2$ | |

Tabelle 1 (Fortsetzung)

| No. | T | Z |
|---|---|---|
| 1025 | Cl | $NH_2$ |
| 1026 | Br | $COSC_2H_5$ |
| 1027 | Br | $COOCH_2CH=CH_2$ |
| 1028 | Cl | $COOCH(CH_3)_2$ |
| 1029 | F | $COOCH(CH_3)_2$ |
| 1030 | Cl | $COOCH_3$ |
| 1031 | Cl | $COOC_2H_5$ |
| 1032 | Cl | $COOC_3H_7n$ |
| 1033 | Cl | $COSCH_3$ |
| 1034 | Cl | $COSC_2H_5$ |
| 1035 | Cl | $COSC_3H_7n$ |
| 1036 | Cl | $COSCH(CH_3)_2$ |
| 1037 | Cl | $COSC_4H_9n$ |
| 1038 | F | $COOCH_3$ |

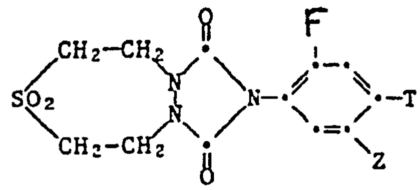

Tabelle 2:

| No. | T | Z | |
|---|---|---|---|
| 2.001 | Cl | $OCHF_2$ | Smp. 232 - 234° |
| 2.002 | Cl | $OCF_2CHClF$ | |
| 2.003 | Cl | $OCH_2CCl{=}CH_2$ | |
| 2.004 | Cl | $OCF_3$ | |
| 2.005 | Cl | $SCF_3$ | |
| 2.006 | Cl | $SCHF_2$ | |
| 2.007 | Cl | $OCF_2CHF_2$ | Smp. 225 - 227° |
| 2.008 | Cl | $OCCl{=}CHCl$ | |
| 2.009 | Cl | $OCH_2{\equiv}CI$ | |
| 2.010 | Cl | $OCH_2CCl{=}CHCl$ | |
| 2.011 | Cl | $OCH(CH_3)CH_2Cl$ | |
| 2.012 | Cl | $OCH_2CH_2F$ | |
| 2.013 | Cl | $OCH_2CH_2Cl$ | |
| 2.014 | Cl | $OCH_2CH_2Br$ | |
| 2.015 | Cl | $OCH_2CH_2I$ | |
| 2.016 | Cl | $OCF_2CHFCF_3$ | Smp. 144 - 147° |
| 2.017 | Cl | $OCF_2CHCl_2$ | |
| 2.018 | Cl | $OCF_2CF_3$ | Smp. 205 - 207° |
| 2.019 | Cl | $OCClF_2$ | |

Tabelle 3: (Fortsetzung)

| No. | T | Z |
|---|---|---|
| 3.018 | Cl | $COSCH(CH_3)COOCH_3$ |
| 3.019 | Cl | $COSCH(CH_3)COOC_2H_5$ |
| 3.020 | Cl | $COSCH(CH_3)COOC_3H_7$-n |
| 3.021 | Cl | $COSCH(CH_3)COOCH(CH_3)_2$ |
| 3.022 | Cl | $COSCH(CH_3)COOC_4H_9$-n |
| 3.023 | Cl | $COSCH(CH_3)COOCH_2CH(CH_3)_2$ |
| 3.024 | Cl | $COSCH(CH_3)COOCH(CH_3)C_2H_5$ |
| 3.025 | Cl | $COSCH(CH_3)COOC(CH_3)_2$ |
| 3.026 | Cl | $COSCH(CH_3)COOCH_2CH{=}CH_2$ |
| 3.027 | Cl | $COSCH(CH_3)COOCHC{\equiv}CH$ |
| 3.028 | Cl | $COSCH(CH_3)$COOCyclohexyl |

Tabelle 3: (Fortsetzung)

| No. | T | Z |
|---|---|---|
| 3.029 | Br | $COOCH_2COOCH_3$ |
| 3.030 | Br | $COOCH_2COOC_2H_5$ |
| 3.031 | Br | $COOCH(CH_3)COOCH_3$ |
| 3.032 | Br | $COOCH(CH_3)COOCH(CH_3)_2$ |
| 3.033 | Br | $COOCH(CH_3)COOC_2H_5$ |
| 3.034 | Br | $COOCH(CH_3)COOCH_2CH{=}CH_2$ |
| 3.035 | Br | $COSCH_2COOCH_3$ |
| 3.036 | Br | $COSCH(CH_3)COOCH_3$ |
| 3.037 | Br | $COSCH(CH_3)COOC_2H_5$ |
| 3.038 | Br | $COSCH_2COOCH(CH_3)_2$ |
| 3.039 | Br | $COSCH(CH_3)COOCH_2CH{=}CH_2$ |
| 3.040 | Br | $COSCH(CH_3)$COOCyclohexyl |

Tabelle 3: (Fortsetzung)

| No. | T | Z |
|---|---|---|
| 3.029 | Br | $COOCH_2COOCH_3$ |
| 3.030 | Br | $COOCH_2COOC_2H_5$ |
| 3.031 | Br | $COOCH(CH_3)COOCH_3$ |
| 3.032 | Br | $COOCH(CH_3)COOCH(CH_3)_2$ |
| 3.033 | Br | $COOCH(CH_3)COOC_2H_5$ |
| 3.034 | Br | $COOCH(CH_3)COOCH_2CH=CH_2$ |
| 3.035 | Br | $COSCH_2COOCH_3$ |
| 3.036 | Br | $COSCH(CH_3)COOCH_3$ |
| 3.037 | Br | $COSCH(CH_3)COOC_2H_5$ |
| 3.038 | Br | $COSCH_2COOCH(CH_3)_2$ |
| 3.039 | Br | $COSCH(CH_3)COOCH_2CH=CH_2$ |
| 3.040 | Br | $COSCH(CH_3)$COOCyclohexyl |

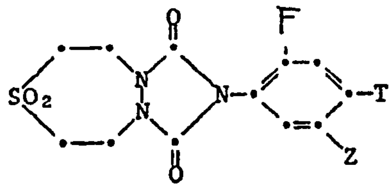

Tabelle 4:

| No. | T | Z |
|---|---|---|
| 4.001 | Cl | $OCH_2COOH$ |
| 4.002 | Cl | $OCH_2COOCH_3$ |
| 4.003 | Cl | $OCH_2COOC_2H_5$ |
| 4.004 | Cl | $OCH_2COOC_3H_7$-n |
| 4.005 | Cl | $OCH_2COOCH(CH_3)_2$ |
| 4.006 | Cl | $OCH_2COOC_4H_9$-n |
| 4.007 | Cl | $OCH_2COOCH_2CH(CH_3)_2$ |
| 4.008 | Cl | $OCH_2COOCH_2CH(CH_3)C_2H_5$ |
| 4.009 | Cl | $OCH_2COOC(CH_3)_3$ |
| 4.010 | Cl | $OCH_2COO$Cyclohexyl |
| 4.011 | Cl | $OCH_2COO$Cyclopentyl |
| 4.012 | Cl | $OCH_2COOCH_2CH{=}CH_2$ |
| 4.013 | Cl | $OCH_2COOCH_2C{\equiv}CH$ |
| 4.014 | Cl | $OCH_2COOCH_2CH_2Cl$ |
| 4.015 | Cl | $OCH_2COOCH_2CH_2OCH_3$ |
| 4.016 | Cl | $OCH_2COOCH_2CCl{=}CH_2$ |
| 4.017 | Cl | $OCH(CH_3)COOH$ |
| 4.018 | Cl | $OCH(CH_3)COOCH_3$ |
| 4.019 | Cl | $OCH(CH_3)COOC_2H_5$ |
| 4.020 | Cl | $OCH(CH_3)COOC_3H_7$-n |
| 4.021 | Cl | $OCH(CH_3)COOCH(CH_3)_2$ |
| 4.022 | Cl | $OCH(CH_3)COOC_4H_9$-n |
| 4.023 | Cl | $OCH(CH_3)COOCH_2CH(CH_3)_2$ |
| 4.024 | Cl | $OCH(CH_3)COOCH_2CH(CH_3)C_2H_5$ |
| 4.025 | Cl | $OCH(CH_3)COOC(CH_3)_3$ |
| 4.026 | Cl | $OCH(CH_3)COO$Cyclohexyl |

Tabelle 4: (Fortsetzung)

| No. | T | Z |
|---|---|---|
| 4.027 | Cl | $OCH(CH_3)COO$Cyclopentyl |
| 4.028 | Cl | $OCH(CH_3)COOCH_2CH{=}CH_2$ |
| 4.029 | Cl | $OCH(CH_3)COOCH_2C{\equiv}CH$ |
| 4.030 | Cl | $OCH(CH_3)COOCH_2CH_2Cl$ |
| 4.031 | Cl | $OCH(CH_3)COOCH_2CH_2OCH_3$ |
| 4.032 | Cl | $OCH(CH_3)COOCH_2CCl{=}CH_2$ |
| 4.033 | Br | $OCH_2COOH$ |
| 4.034 | Br | $OCH_2COOCH_3$ |
| 4.035 | Br | $OCH_2COOC_2H_5$ |
| 4.036 | Br | $OCH_2COOCH(CH_3)_2$ |
| 4.037 | Br | $OCH_2COOC_4H_9$-n |
| 4.038 | Br | $OCH_2COOCH_2CH{=}CH_2$ |
| 4.039 | Br | $OCH(CH_3)COOH$ |
| 4.040 | Br | $OCH(CH_3)COOCH_3$ |
| 4.041 | Br | $OCH(CH_3)COOC_2H_5$ |
| 4.042 | Br | $OCH(CH_3)COOCH(CH_3)_2$ |
| 4.043 | Br | $OCH(CH_3)COOC_4H_9$-n |
| 4.044 | Br | $OCH(CH_3)COOCH_2CH{=}CH_2$ |
| 4.045 | Br | $OCH(CH_3)COO$Cyclopentyl |
| 4.046 | Br | $OCH(C_2H_5)COOH$ |
| 4.047 | Br | $OCH(C_2H_5)COOCH_3$ |
| 4.048 | Br | $OCH(C_2H_5)COOC_2H_5$ |
| 4.049 | Br | $OCH(C_2H_5)COOCH(CH_3)_2$ |
| 4.050 | Br | $OCH(C_2H_5)COOC_4H_9$-n |
| 4.051 | Br | $OCH(C_2H_5)COOCH_2CH{=}CH_2$ |
| 4.052 | Br | $OCH(C_2H_5)COO$Cyclohexyl |

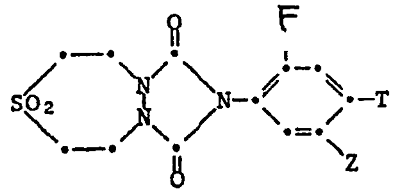

Tabelle 5:

| No. | T | Z |
|---|---|---|
| 5.001 | Cl | $SCH_2COOH$ |
| 5.002 | Cl | $SCH_2COOCH_3$ |
| 5.003 | Cl | $SCH_2COOC_2H_5$ |
| 5.004 | Cl | $SCH_2COOC_3H_7$-n |
| 5.005 | Cl | $SCH_2COOCH(CH_3)_2$ |
| 5.006 | Cl | $SCH_2COOC_4H_9$-n |
| 5.007 | Cl | $SCH_2COOCH_2CH(CH_3)_2$ |
| 5.008 | Cl | $SCH_2COOCH_2CH(CH_3)C_2H_5$ |
| 5.009 | Cl | $SCH_2COOC(CH_3)_3$ |
| 5.010 | Cl | $SCH_2COO$Cyclohexyl |
| 5.011 | Cl | $SCH_2COO$Cyclopentyl |
| 5.012 | Cl | $SCH_2COOCH_2CH{=}CH_2$ |
| 5.013 | Cl | $SCH_2COOCH_2C{\equiv}CH$ |
| 5.014 | Cl | $SCH_2COOCH_2CH_2Cl$ |
| 5.015 | Cl | $SCH_2COOCH_2CH_2OCH_3$ |
| 5.016 | Cl | $SCH_2COOCH_2CCl{=}CH_2$ |
| 5.017 | Cl | $SCH(CH_3)COOH$ |
| 5.018 | Cl | $SCH(CH_3)COOCH_3$ |
| 5.019 | Cl | $SCH(CH_3)COOC_2H_5$ |
| 5.020 | Cl | $SCH(CH_3)COOC_3H_7$-n |
| 5.021 | Cl | $SCH(CH_3)COOCH(CH_3)_2$ |
| 5.022 | Cl | $SCH(CH_3)COOC_4H_9$-n |
| 5.023 | Cl | $SCH(CH_3)COOCH_2CH(CH_3)_2$ |
| 5.024 | Cl | $SCH(CH_3)COOCH_2CH(CH_3)C_2H_5$ |
| 5.025 | Cl | $SCH(CH_3)COOC(CH_3)_3$ |
| 5.026 | Cl | $SCH(CH_3)COO$Cyclohexyl |

Tabelle 5: (Fortsetzung)

| No. | T | Z |
|---|---|---|
| 5.027 | Cl | $SCH(CH_3)COO$Cyclopentyl |
| 5.028 | Cl | $SCH(CH_3)COOCH_2CH=CH_2$ |
| 5.029 | Cl | $SCH(CH_3)COOCH_2C\equiv CH$ |
| 5.030 | Cl | $SCH(CH_3)COOCH_2CH_2Cl$ |
| 5.031 | Cl | $SCH(CH_3)COOCH_2CH_2OCH_3$ |
| 5.032 | Cl | $SCH(CH_3)COOCH_2CCl=CH_2$ |
| 5.033 | Br | $SCH_2COOH$ |
| 5.034 | Br | $SCH_2COOCH_3$ |
| 5.035 | Br | $SCH_2COOC_2H_5$ |
| 5.036 | Br | $SCH_2COOCH(CH_3)_2$ |
| 5.037 | Br | $SCH_2COOC_4H_9$-n |
| 5.038 | Br | $SCH_2COOCH_2CH=CH_2$ |
| 5.039 | Br | $SCH(CH_3)COOH$ |
| 5.040 | Br | $SCH(CH_3)COOCH_3$ |
| 5.041 | Br | $SCH(CH_3)COOC_2H_5$ |
| 5.042 | Br | $SCH(CH_3)COOCH(CH_3)_2$ |
| 5.043 | Br | $SCH(CH_3)COOC_4H_9$-n |
| 5.044 | Br | $SCH(CH_3)COOCH_2CH=CH_2$ |
| 5.045 | Br | $SCH(CH_3)COO$Cyclopentyl |
| 5.046 | Br | $SCH(C_2H_5)COOH$ |
| 5.047 | Br | $SCH(C_2H_5)COOCH_3$ |
| 5.048 | Br | $SCH(C_2H_5)COOC_2H_5$ |
| 5.049 | Br | $SCH(C_2H_5)COOCH(CH_3)_2$ |
| 5.050 | Br | $SCH(C_2H_5)COOC_4H_9$-n |
| 5.051 | Br | $SCH(C_2H_5)COOCH_2CH=CH_2$ |
| 5.052 | Br | $SCH(C_2H_5)COO$Cyclohexyl |

Formulierungsbeispiele:

Beispiel 5: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 bis 5 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 bis 5 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 bis 5 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabelle 1 bis 5 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabelle 1 bis 5 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabelle 1 bis 5 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff gemäss Tabelle 1 bis 5 | 5 % |
| Isopropylamin | 1 % |
| Octylphenolpolyäthylenglykoläther (78 Mol AeO) | 3 % |
| Wasser | 91 % |

Biologische Beispiele:

Beispiel 6: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem Emulsionskonzentrat behandelt. Es werden Konzentrationen von 4 kg Wirksubstanz/Hektar angewendet. Die Saatschalen werden im Gewächshaus bei 22-25°C und 50-70 % relativer Luftfeuchtigkeit gehalten und der Versuch nach 3 Wochen abgeschlossen und die Resultate nach folgender Notenskala bewertet.

1 Pflanze hat nicht gekeimt oder ist eingegangen
2-3 sehr starke Schäden
4 starke Schäden
5 mittlere Schäden, die Pflanzen sind verkümmert
6 Schäden, die Pflanze kann regenerieren
7-8 leichte Schäden
9 normales Wachstum, wie das unbehandelter Pflanzen.

Die Resultate sind unten zusammengefasst.

Die Verbindung A ist das aus der DOS-2 638 543 bekannte 9-(2,5-Diäthoxyphenyl)-8,10-dioxo-4,1,7,9-thiatriazabicylco[5.3.0]-decan-4,4-dioxid

| Verbindung | Avena sativa | Sinapis alba | Setaria italica | Stellaria media |
|---|---|---|---|---|
| 1.001 | 1 | 1 | 1 | 1 |
| A | 9 | 9 | 9 | 9 |

Beispiel 7: Post-emergente Herbizid-Wirkung (Kontaktherbizid)

Eine Anzahl Pflanzen, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Die Resultate sind unten zusammengefasst

| Pflanze | Verbindung 1.001 | A |
|---|---|---|
| Avena fatua | 1 | 9 |
| Setaria italica | 1 | 9 |
| Lolium perenne | 1 | 9 |
| Sotanum lycopers. | 1 | 9 |
| Sinapis alba | 1 | 7 |
| Stellaria media | 2 | 9 |
| Phaseolus vulgaris | 1 | 8 |

Beispiel 8: Herbizidwirkung vor dem Auflaufen der Pflanzen

Kunststofföpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4

bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird der Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet. Die geprüften Verbindungen der Tabellen 1 bis 5 zeigen dabei gute bis sehr gute Herbizidwirkung.

Beispiel 9: Herbizidwirkung für Wasserreis (paddy rice)

Die Wasserunkräuter Echinochloa crus galli und Monocharia vag. werden in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 bis 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die geprüften Verbindungen der Tabellen 1 bis 5 schädigen dabei die Unkräuter, nicht aber den Reis.

Beispiel 10: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen der Tabellen 1 bis 5 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 11: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die geprüften Wirkstoffe der Tabellen 1 bis 5 eine merkliche Erhöhung der Anzahl und des Gewichts der Schoten im Haupttrieb.

Beispiel 12: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabellen 1 bis 5 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 13: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgeleufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1 bis 5 besprüht. Die Wirkstoffmenge beträgt umgerechnet bis zu 500 g Aktivsubstanz pro Hektar.

21 Tage nach Applikation wird das Wachstum der Gräser beurteilt.

Die geprüften Verbindungen der Tabellen 1 bis 4 bewirken eine Reduzierung des Neuzuwachses von um 10-30 % im Vergleich zur unbehandelten Kontrolle.

Beispiel 14: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter). In diesem Versuch liessen die geprüften Verbindungen der Tabellen 1 bis 5 bei Aufwandmengen von 0,6 und 1,2 kg/ha nach 7 Tagen bloss noch wenige vertrocknete Blätter auf den Pflanzen (über 80 % Blattfall und Austrocknung).

**Patentansprüche**

1. Neue anellierte Triazol-Verbindungen der Formel I

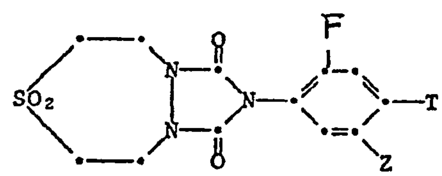

(I)

worin

T Halogen und

Z einen Rest -XR oder -COXR

X Sauerstoff, Schwefel oder den Rest $-NR_1-$

R Wasserstoff, einen $C_1$-$C_6$-Alkyl-, $C_3$-$C_6$-Cycloalkyl-, einen über ein gesättigte Kohlenstoffatom gebundenen $C_3$-$C_6$-Alkenyl- oder $C_3$-$C_6$-Alkinylrest, der unsubstituiert oder durch Halogen substituiert ist, oder einen Rest $-A-COXR_1$ wobei

A eine $C_1$-$C_4$ Alkylenbrücke bedeutet und

$R_1$ Wasserstoff oder $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeuten.

2. Verbindungen gemäss Anspruch 1 der Formel I, worin

T Fluor, Chlor oder Brom bedeuten während

Z die im Anspruch 1 gegebene Bedeutung hat.

3. Verbindungen gemäss Anspruch 1 der Formel I, worin

T Chlor oder Brom,

Z einen Rest -XR oder -COXR

X Sauerstoff, Schwefel, den Rest $-NR_1-$

R Wasserstoff, $C_1$-$C_4$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl und

$R_1$ Wasserstoff $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeuten.

4. Verbindungen gemäss Anspruch 1 der Formel I, worin

T Chlor oder Brom

Z einen Rest -XR,

X Sauerstoff oder Schwefel,

R Wasserstoff, einen $C_1$-$C_6$ Alkyl-, $C_3$-$C_6$ Cycloalkyl-, einen über ein gesättigtes Kohlenstoffatom gebundenen $C_3$-$C_6$ Alkenyl- oder $C_3$-$C_6$ Alkinylrest, der unsubstituiert oder durch Halogen substituiert ist bedeuten.

5. Verbindungen gemäss Anspruch 1 der Formel I, worin

T Chlor oder Brom

Z einen Rest -XR

X einen Rest $-NR_1-$ und

R Wasserstoff, $C_1$-$C_6$-Alkyl oder über ein gesättigtes Kohlenstoffatom gebundenes $C_3$-$C_6$-Alkenyl und

$R_1$ Wasserstoff oder $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl bedeutet.

6. Verbindungen gemäss Anspruch 1 der Formel I, worin

T Chlor oder Brom,

Z einen Rest -COXR

X Sauerstoff oder Schwefel

R Wasserstoff, $C_1$-$C_6$ Alkyl, $C_3$-$C_6$ Cycloalkyl, ein über ein gesättigtes Kohlenstoffatom gebundenes $C_3$-$C_6$ Alkenyl oder $C_3$-$C_6$ Alkinyl bedeuten.

7. Verbindungen gemäss Anspruch 1 der Formel I, worin

T Chlor oder Brom

Z einen Rest -XR

R einen Rest $-A-COXR_1$ und

A eine $C_1$-$C_4$ Alkylenbrücke

$R_1$ Wasserstoff oder $C_1$-$C_4$ Alkyl, $C_3$-$C_6$ Cycloalkyl, ein über ein gesättigtes Kohlenstoffatom gebundenes $C_3$-$C_4$ Alkenyl oder $C_3$-$C_4$ Alkinyl und

X Sauerstoff oder Schwefel bedeuten.

8. 9-(2-Fluor-4-chlor-5-isopropyloxy-phenyl)-8,10-dioxo-4,1,7,9-thiatriazabicyclo[5.3.0]-decan-4,4-dioxid gemäss Anspruch 1.

9. Verfahren zur Herstellung der Verbindungen der Formel I gemäss Anspruch 1, dadurch gekennzeichnet, dass man ein 1-(2-Phenyl)-1H-1,3,4-triazolidin-2,5-dion der Formel II

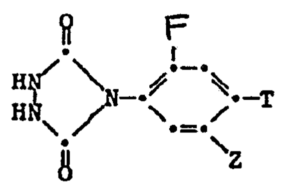

II

worin T und Z die unter der Formel I gegebene Bedeutung haben in einem inerten organischen Lösungsmittel bei einer Temperatur von 0° bis 150°C in Gegenwart der Katalytischen Menge einer Base mit Divinylsulfon $(CH_2{=}CH)_2SO_2$ kondensiert.

10. Ein herbizides und Pflanzenwachstum regulierendes Mittel, dadurch gekennzeichnet, dass es neben Träger- und/oder anderen Zuschlagstoffen als Wirkstoff eine anellierte Triazol-Verbindung der Formel I, Anspruch 1 enthält.

11. Die Verwendung eines Wirkstoffes gemäss Anspruch 1, oder einen solchen Wirkstoff enthaltendes Mittel zur Bekämpfung unerwünschten Pflanzenwachstums.

12. Die Verwendung eines Wirkstoffes gemäss Anspruch 1 oder einen solchen Wirkstoff enthaltendes Mittel zur Hemmung des Pflanzenwachstums.

13. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Wirkstoffes der Formel I, Anspruch 1 oder eines eine solche Verbindung enthaltenden Mittels behandelt.

14. Verfahren zur Unterdrückung des Pflanzenwachstums über das 2-Blattstadium hinaus, dadurch gekennzeichnet, dass man die Pflanzen während ihres Wachstums mit einer wirksamen Menge eines Wirkstoffes der Formel I Anspruch 1 oder eines einen solchen Wirkstoff enthaltenden Mittels behandelt.

## Claims

1. A novel fused triazole compound of the formula I

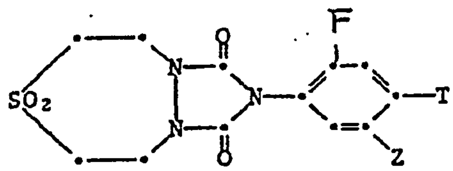

(I)

in which

T is halogen,

Z is a radical -XR or -COXR,

X is oxygen, sulfur or the radical $-NR_1-$,

R is hydrogen, a $C_1$-$C_6$alkyl or $C_3$-$C_6$cycloalkyl radical; a $C_3$-$C_6$alkenyl or $C_3$-$C_6$alkynyl radical which is linked via a saturated carbon atom, and each of which is unsubstituted or substituted by halogen, or is a radical $-A-COXR_1$ in which

A is a $C_1$-$C_4$alkylene bridge and

$R_1$ is hydrogen or $C_1$-$C_4$alkyl or $C_3$-$C_6$cycloalkyl, or is $C_3$-$C_4$alkenyl or $C_3$-$C_4$alkynyl which is linked via a saturated carbon atom.

2. A compound according to claim 1 of the formula I in which

T is fluorine, chlorine or bromine and

Z has the meaning given in claim 1.

3. A compound according to claim 1 of the formula I in which

T is chlorine or bromine,

Z is a radical -XR or -COXR,

X is oxygen, sulfur or the radical $-NR_1-$,

R is hydrogen, $C_1$-$C_4$alkyl or $C_3$-$C_6$cycloalkyl, or $C_3$-$C_6$alkenyl or $C_3$-$C_6$alkynyl which is linked via a saturated carbon atom and

$R_1$ is hydrogen, $C_1$-$C_4$alkyl or $C_3$-$C_6$cycloalkyl, or is $C_3$-$C_4$alkenyl or $C_3$-$C_4$alkynyl which is linked via a saturated carbon atom.

4. A compound according to claim 1 of the formula I in which

T is chlorine or bromine,

Z is a radical -XR,

X is oxygen or sulfur and

R is hydrogen, a $C_1$-$C_6$alkyl or $C_3$-$C_6$cycloalkyl radical, or a $C_3$-$C_6$alkenyl or $C_3$-$C_6$alkynyl radical which is linked via a saturated carbon atom, and each of which is unsubstituted or substituted by halogen.

5. A compound according to claim 1 of the formula I in which

T is chlorine or bromine,

Z is a radical -XR,

X is a radical $-NR_1-$ and

R is hydrogen, $C_1$-$C_6$alkyl, or $C_3$-$C_6$alkenyl which is linked via a saturated carbon atom and $R_1$ is hydrogen or $C_1$-$C_4$alkyl or $C_3$-$C_6$cycloalkyl, or is $C_3$-$C_4$alkenyl or $C_3$-$C_4$alkynyl which is linked via a saturated carbon atom.

6. A compound according to claim 1 of the formula I in which

T is chlorine or bromine,

Z is a radical -COXR,

X is oxygen or sulfur and

R is hydrogen, $C_1$-$C_6$alkyl or $C_3$-$C_6$cycloalkyl, or is $C_3$-$C_6$alkenyl or $C_3$-$C_6$alkynyl which is linked via a saturated carbon atom.

7. A compound according to claim 1 of the formula I in which

T is chlorine or bromine,

Z is a radical -XR,

R is a radical $-A-COXR_1$ and

A is a $C_1$-$C_4$alkylene bridge,

$R_1$ is hydrogen or $C_1$-$C_4$alkyl or $C_3$-$C_6$cycloalkyl, or is $C_3$-$C_4$alkenyl or $C_3$-$C_4$alkynyl which is linked via a saturated carbon atom and

X is oxygen or sulfur.

8. 9-(2-Fluoro-4-chloro-5-isopropyloxyphenyl)-8,10-dioxo-4,1,7,9-thiatriazabicyclo[5.3.0]decane-4,4-dioxide according to claim 1.

9. A process for producing a compound of the formula I according to claim 1, which comprises subjecting

a 1-(2-phenyl)-1H-1,3,4-triazolidine-2,5-dione of the formula II

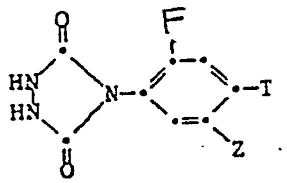

(II),

in which T and Z have the meanings given under the formula I, in an inert organic solvent at a temperature of 0° to 150°C in the presence of the catalytic amount of a base as catalyst to a condensation reaction with divinylsulfone, $(CH_2{=}CH)_2SO_2$.

10. A herbicidal and plant-growth regulating composition which contains as active substance a fused triazole compound of the formula I, claim 1, together with carriers and/or other additives.

11. The use of an active substance according to claim 1, or a composition containing such an active substance, for controlling unwanted plant growth.

12. The use of an active substance according to claim 1, or a composition containing such an active substance, for inhibiting plant growth.

13. A method for the selective pre- or postemergence control of weeds in crops of useful plants, which comprises treating the crops of useful plants or the cultivated area thereof with an effective amount of an active substance of the formula I, claim 1, or of a composition containing such a compound.

14. A method of suppressing plant growth beyond the 2-leaf stage, which comprises treating the plants during their growth with an effective amount of an active substance of the formula I, claim 1, or of a composition containing such an active substance.

**Revendications**

1. Nouveaux dérivés du triazole à cycles condensés, répondant à la formule I

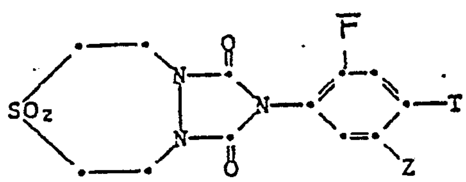

(I)

dans laquelle

T représente un halogène, et

Z un groupe -XR ou -COXR,

X représente l'oxygène, le soufre ou le groupe $-NR_1-$,

R représente l'hydrogène, un groupe alkyle en C1-C6, cycloalkyle en C3-C6, un groupe alcényle en C3-C6 ou alcynyle en C3-C6 relié par l'intermédiaire d'un atome de carbone saturé, non substitué ou substitué par des halogènes, ou bien un groupe $-A-COXR_1$ dans lequel

A représente un pont alkylène en C1-C4, et

$R_1$ représente l'hydrogène ou un groupe alkyle en C1-C4, cycloalkyle en C3-C6, un groupe alcényle en C3-C4 ou alcynyle en C3-C4 relié par l'intermédiaire d'un atome de carbone saturé.

2. Composés selon la revendication 1, de formule I dans laquelle

T représente le fluor, le chlore ou le brome, et

Z a les significations indiquées dans la revendication 1.

3. Composés selon la revendication 1, de formule I dans laquelle

T représente le chlore ou le brome,

Z représente un groupe -XR ou -COXR,

X représente l'oxygène, le soufre, le groupe $-NR_1-$,

R représente l'hydrogène, un groupe alkyle en C1-C4, cycloalkyle en C3-C6, un groupe alcényle en C3-C6 ou alcynyle en C3-C6 relié par l'intermédiaire d'un atome de carbone saturé, et

$R_1$ représente l'hydrogène, un groupe alkyle en C1-C4, cycloalkyle en C3-C6, un groupe alcényle en C3-C4 ou alcynyle en C3-C4 relié par l'intermédiaire d'un atome de carbone saturé.

4. Composés selon la revendication 1, de formule I dans laquelle
T représente le chlore ou le brome,
Z représente un groupe -XR,
X représente l'oxygène ou le soufre,
R représente l'hydrogène, un groupe alkyle en C1-C6, cycloalkyle en C3-C6, un groupe alcényle en C3-C6 ou alcynyle en C3-C6 relié par l'intermédiaire d'un atome de carbone saturé, non substitué ou substitué par des halogènes.

5. Composés selon la revendication 1, de formule I dans laquelle
T représente le chlore ou le brome,
Z représente un groupe -XR,
X représente un groupe $-NR_1-$, et
R représente l'hydrogène, un groupe alkyle en C1-C6 ou un groupe alcényle en C3-C6 relié par l'intermédiaire d'un atome de carbone saturé, et $R_1$ représente l'hydrogène ou un groupe alkyle en C1-C4, cycloalkyle en C3-C6, un groupe alcényle en C3-C4 ou alcynyle en C3-C4 relié par l'intermédiaire d'un atome de carbone saturé.

6. Composés selon la revendication 1, de formule I dans laquelle
T représente le chlore ou le brome,
Z représente un groupe -COXR,
X représente l'oxygène ou le soufre,
R représente l'hydrogène, un groupe alkyle en C1-C6, cycloalkyle en C3-C6, un groupe alcényle en C3-C6 ou alcynyle en C3-C6 relié par l'intermédiaire d'un atome de carbone saturé.

7. Composés selon la revendication 1, de formule I dans laquelle
T représente le chlore ou le brome,
Z représente un groupe -XR,
R représente un groupe $-A-COXR_1$, et
A représente un pont alkylène en C1-C4,
$R_1$ représente l'hydrogène ou un groupe alkyle en C1-C4, cycloalkyle en C3-C6, un groupe alcényle en C3-C4 ou alcynyle en C3-C4 relié par l'intermédiaire d'un atome de carbone saturé, et
X représente l'oxygène ou le soufre.

8. Le 4,4-dioxyde du 9-(2-fluoro-4-chloro-5-isopropyloxy-phényl)-8,10-dioxo-4,1,7,9-thiatriazabicyclo [5.3.0]décane selon la revendication 1.

9. Procédé de préparation des composés de formule I de la revendication 1, caractérisé en ce que l'on condense une 1-(2-phényl)-1H-1,3,4-triazolidine-2,5-dione de formule II

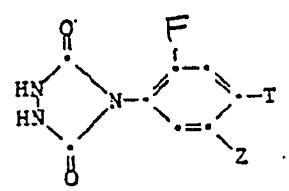

II

dans laquelle T et Z ont les significations indiquées en référence à la formule I, dans un solvant organique inerte, à une température de 0 à 150°C, en présence de la quantité catalytique d'une base, avec la divinylsulfone $(CH_2=CH)_2SO_2$.

10. Un produit herbicide et régulateur de la croissance des végétaux, caractérisé en ce qu'il contient en tant que substance active, avec des véhicules et/ou d'autres additifs, un dérivé du triazole à cycles condensés de formule I, revendication 1.

11. L'utilisation d'une substance active selon revendication 1 ou d'un produit contenant une telle substance active pour combattre les croissances de végétaux indésirables.

12. L'utilisation d'une substance active selon revendication 1 ou d'un produit contenant une telle substance active pour l'inhibition de la croissance des végétaux.

13. Procédé pour combattre sélectivement en pré-levée ou en post-levée les végétaux adventices dans les cultures de végétaux utiles, caractérisé en ce que l'on traite les cultures de végétaux utiles ou leur aire de culture par une quantité efficace d'une substance active de formule I, revendication 1, ou d'un produit contenant un tel composé.

14. Procédé pour inhiber la croissance de végétaux au-delà du stade deux feuilles, caractérisé en ce que l'on traite les végétaux en cours de croissance par une quantité efficace d'une substance active de formule I, revendication 1, ou d'un produit contenant une telle substance active.